(19) Europäisches Patentamt
European Patent Office
Office européen des brevets

(11) Veröffentlichungsnummer: **0 450 176 A1**

# (12) EUROPÄISCHE PATENTANMELDUNG

(21) Anmeldenummer: **90124695.9**

(22) Anmeldetag: **19.12.90**

(51) Int. Cl.5: **C08G 18/32**, C08G 18/64, A61K 9/20, A61K 9/32, A61K 9/50, A61K 9/16

(30) Priorität: **02.03.90 DE 4006521**

(43) Veröffentlichungstag der Anmeldung: **09.10.91 Patentblatt 91/41**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Müller, Hanns-Peter, Dr.**
**Weizenfeld 36**
**W-5060 Bergisch Gladbach 2(DE)**
Erfinder: **Bauer, Kurt Heinz, Prof.Dr.**
**Im Finkeler 4**
**W-7800 Freiburg-Tiengen(DE)**

## (54) Zuckerhaltige Polymere zur Umhüllung und Einbettung von Arzneistoffen.

(57) Die vorliegende Erfindung betrifft neue zuckerhaltige Polymere zur Umhüllung und/oder Einbettung von oral applizierbaren pharmazeutischen Wirkstoffen, enthaltend Di- und/oder Polysaccharidsegmente, die dazu führen, daß die Wirkstoffe, die in den Polymeren enthalten sind, erst im Dickdarm freigegeben werden, ihre Herstellung und ihre Verwendung bei der Herstellung von Arzneimitteln sowie die so umhüllten und/oder eingebetteten Wirkstoffe.

EP 0 450 176 A1

Die vorliegende Erfindung betrifft neue zuckerhaltige Polymere zur Umhüllung und/oder Einbettung von oral applizierbaren pharmazeutischen Wirkstoffen, enthaltend Di- und/oder Polysaccharidsegmente, die dazu führen, daß die Wirkstoffe, die in den Polymeren enthalten sind, erst im Dickdarm freigegeben werden, ihre Herstellung und ihre Verwendung bei der Herstellung von Arzneimitteln sowie die so umhüllten und/oder eingebetteten Wirkstoffe.

Von besonderem Interesse sind Block-Copolymere, die über Urethan- und/oder Harnstoff-Strukturen aufgebaut sind und die als Saccharidsegmente lineare oder verzweigte Kohlehydrate des Molmassenbereichs bis ca. $10^5$ enthalten, wobei diese Di- oder Polysaccharidsegmente bevorzugt aus Pyranoseeinheiten aufgebaut sind. Besonders geeignet sind Polymere, deren Saccharidsegmente die substituierten Pyranoseeinheiten enthalten mit einem durchschnittlichen Substitutionsgrad pro Pyranoseeinheit von bis zu 3, insbesondere von 2,5 bis 3. Als Substituenten der Pyranosesegmente seien bevorzugt aliphatische, araliphatische und/oder aromatische Ether, Ester und/oder Carbamate genannt. Der Aufbau der erfindungsgemäßen Polymeren erfolgt bevorzugt unter Verwendung von aliphatischen, araliphatischen und/oder aromatischen Polyisocyanaten.

Die erfindungsgemäßen Polymere sind neu. Zwar ist die Herstellung von zuckerhaltigen Polyurethanen durch Einsatz von Prepolymeren bekannt. Dabei wurden bisher jedoch nur glucosehaltige Komponenten eingesetzt, was zu Polyurethanen führte, welche die erwünschten Eigenschaften bezüglich Löslichkeit, Quellung und Abbaubarkeit vor allem im Dickdarm nicht besitzen.

Bisher bekannte Arzneiformen zur Freisetzung von Wirkstoffen im Dickdarm, bei denen die Arzneistoffe oder Arzneistoffkerne mit Polymeren umhüllt oder in Polymere eingebettet waren, erreichen dieses Ziel nicht oder nur mangelhaft. Die hierfür verwendeten Filmüberzüge oder Einbettungsmaterialien bestehen aus herkömmlichen Filmbildnern, welche durch Vernetzung mit Azogruppen modifiziert sind (Saffran et al., U.S. Pat. 4 663 308) oder aus Mischungen derselben [Rubinstein et al., J. Pharm. 30, 95 bis 99 (1986)].

Die Arbeitsgruppe um Saffran polymerisierte Methacrylate mit verschiedenen Monomeren wie z.B. Styrol und einer polymerisationsfähigen Azokomponente z.B. Divinylazobenzol. Auf diese Weise sollten quervernetzte Polymere entstehen.

Ein Abbau dieser Polymere im Colon konnte auch nicht innerhalb von 8 Tagen nachgewiesen werden. Für den vorgesehenen Verwendungszweck ist jedoch ein Abbau innerhalb von Minuten oder wenigen Stunden zu fordern.

Die Arbeitsgruppe um Rubinstein verwendete Mischungen verschiedener Polymethacrylate, wobei die resultierenden Filmüberzüge ihre Permeabilität oder Löslichkeit in Abhängigkeit des pH-Wertes ändern. Mit diesen Überzügen umhüllte oder in diese Polymere eingebettete Wirkstoffe können nur diffusionskontrolliert freigesetzt werden. Eine diffusionskontrollierte Freisetzung ist jedoch zu langsam, da die zur Verfügung stehende Freisetzung- und Resorptionszeit im Colon limitiert ist. Außerdem ist der Beginn der Freisetzung ungünstigerweise noch von einem Anstieg des pH-Wertes des Darminhaltes abhängig. In dem in Frage kommenden Darmabschnitt ist der pH-Wert jedoch bereits weitgehend konstant. Somit ergibt sich der Zeitpunkt des Freisetzungsbeginns aus der Transportgeschwindigkeit des Darminhaltes.

Solche Filmüberzüge beginnen also bereits im proximalen oder im distalen Ileum zu quellen, um den Wirkstoff dann im Colon ascendens durch den maximal gequollenen Filmüberzug freizusetzen. Eine solche Arzneiform führt bestenfalls zu einer geringen und nicht reproduzierbaren Bioverfügbarkeit.

Kurita et al. [Makromol. Chem. 161, 1861-1870 (1980)] stellten Polyurethane aus D-Cellobiose und verschiedenen Diisocyanaten her. Steinmann (U.S. Pat. 3 386 932) stellte oligo- und polysaccharidhaltige Polyurethane her, indem er Cellulosetriacetat depolymerisierte und es mit Diisocyanaten und Polyesterdiolen versetzte. Gilbert et al. (U.S. Pat. 3 950 282) deacetylierten die nach Steinmann hergestellten Polymere und untersuchten ihre enzymatische Abbaubarkeit.

Die beschriebenen Polymere eignen sich nicht als Filmüberzüge oder Einbettungsmaterialien für die erfindungsgemäße Anwendung; entweder wegen ihrer schlechten Löslichkeit in geeigneten Lösungsmitteln oder wegen ihrem zu hohen Quellungsvermögen im wäßrigen Milieu. Jedenfalls aber sind sie wegen ihrer fehlenden Abbaubarkeit in der Mikroflora des Dickdarmes nicht geeignet.

Es ist daher außerordentlich überraschend, daß erfindungsgemäß filmbildende Polymere, insbesondere Polyurethane, synthetisiert werden können, die einerseits den Gastrointestinaltrakt unbeschadet überstehen, und andererseits im Dickdarm schnell abgebaut werden können. Dies war auch deshalb nicht zu erwarten, weil andere Arbeitsgruppen, beispielsweise Gilbert et al. (U.S. Pat. 3 950 282), eine enzymatische Abbaubarkeit derivatisierter Zucker widerlegten und somit ein zu überwindendes Vorurteil für diese Erfindung darstellten.

Gegenstand der Erfindung sind zuckerhaltige Polymere zur Umhüllung und/oder Einbettung von oral applizierbaren pharmazeutischen Wirkstoffen, enthaltend Di- und/oder Polysaccharidsegmente, die erst im Dickdarm die enthaltenden Wirkstoffe freigeben.

Erfindungsgemäß bevorzugte derartige Polymere und pharmazeutische Zubereitungen sind dadurch gekennzeichnet,

- daß die zuckerhaltigen Polymeren Urethan- und/oder Harnstoff-Strukturen aufweisende Block-Copolymere darstellen,
- daß die zuckerhaltigen Polymeren als Saccharidsegmente lineare oder verzweigte Kohlenhydrate vom Molekulargewicht 340 bis ca. 10.000 enthalten,
- daß in den zuckerhaltigen Polymeren die Di- oder Polysaccharidsegmente als Pyranoseeinheiten vorliegen, wobei pro Pyranoseeinheit bis zu drei OH-Gruppen substituiert sind,
- daß in den zuckerhaltigen Polymeren die Pyranoseeinheiten mit einem Substitutionsgrad von 2,5 bis 3 vorliegen,
- daß in den zuckerhaltigen Polymeren die Pyranoseeinheiten durch aliphatische, araliphatische und/oder aromatische Ether, Ester und/oder Carbamate substituiert sind und
- daß die zuckerhaltigen Polymeren unter Verwendung von aliphatischen, araliphatischen und/oder aromatischen Polyisocyanaten aufgebaut sind.

Die Erfindung betrifft ferner ein Verfahren zur Herstellung von mit Di- und/oder Polysaccharidsegmente aufweisenden zuckerhaltigen Polymeren umhüllte und/oder in solche Polymere eingebettete oral applizierbaren pharmazeutischen Wirkstoffen, dadurch gekennzeichnet, daß man eine überschüssige Menge eines Polyisocyanats mit Di- und/oder Polysaccharidsegmente aufweisenden zuckerhaltigen Diolen, gegebenenfalls im Gemisch mit nichtzuckerhaltigen Polyolen des Molgewichtsbereichs 62 bis 12.000, zu endständigen Isocyanatgruppen aufweisenden Prepolymeren umsetzt, anschließend eine Kettenverlängerung dieser Prepolymeren mit Kettenverlängerungsmitteln durchführt und pharmazeutische Wirkstoffe mit den so erhaltenen Urethan- und/oder Harnstoff-Strukturen aufweisenden zuckerhaltigen Polymeren umhüllt und/oder in diese Polymeren einbettet.

Erfindungsgemäß besonders bevorzugt ist dabei,

- daß man als Polyisocyanate aliphatische und/oder cycloaliphatische Diisocyanate der Formel

  $Q(NCO)_n$

  in der

  n 2 bis 4, vorzugsweise 2, bedeutet und

  Q einen Kohlenwasserstoffrest mit 2 bis 18, vorzugsweise 6 bis 10 C-Atomen, darstellt, verwendet,
- daß man als Polyisocyanate Lysinester-diisocyanate und/oder Lysinester-Triisocyanat verwendet,
- daß man als Polyisocyanate Estergruppen aufweisende aromatische Polyisocyanate mit den Struktureinheiten der Formel

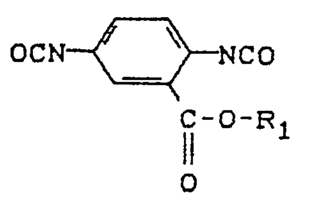

und/oder

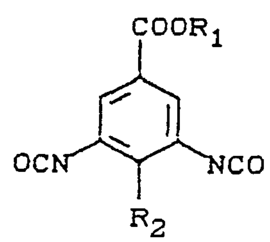

  worin

  $R_1$ einen gegebenenfalls verzweigten $C_1$-$C_{20}$-, vorzugsweise $C_1$-$C_8$-Alkylrest und

  $R_2$ einen $C_1$-$C_{10}$-, vorzugsweise $C_1$-$C_4$-Alkylrest, $C_1$-$C_4$-Alkoxygruppen, vorzugsweise eine $O\text{-}CH_3$-Gruppe oder ein Halogenatom, vorzugsweise ein Chloratom bedeuten, verwendet und
- daß man Mischungen unterschiedlicher Polyisocyanate verwendet.

Die Erfindung betrifft auch die Verwendung von Di- und/oder Polysaccharidsegmente aufweisenden zuckerhaltigen Polymeren zur Herstellung von Filmüberzügen und Einbettungen von oral applizierbaren pharmazeutischen Wirkstoffen sowie oral applizierbare Arzneizubereitungen mit einer Wirkstoff-Freigabe im Dickdarm, dadurch gekennzeichnet, daß die Wirkstoffe von Di- und/oder Polysaccharidsegmente aufweisenden zuckerhaltigen Polymeren umhüllt und/oder in diese eingebettet sind.

Die Herstellung der erfindungsgemäßen Polyurethane erfolgt vorzugsweise nach üblichen Verfahren unter Einsatz von Prepolymeren, d.h. durch Umsetzung einer überschüssigen Menge eines geeigneten

Diisocyanats mit zuckerhaltigen Diolen zu den entsprechenden endständige Isocyanatgruppen aufweisenden Prepolymeren und anschließender Kettenverlängerung dieser Prepolymeren mit verschiedenen Kettenverlängerungsmitteln.

Für die Herstellung der erfindungsgemäßen Polyurethane geeignete zuckerhaltige Diole sind insbesondere solche mit einer aus underivatisierter oder derivatisierter Mannose- und/oder Galactoseeinheiten aufgebauten linearen Kette, mit oder ohne weiteren Verzweigungen, bestehend aus beliebigen Pyranosen, derivatisiert oder underivatisiert.

Die erfindungsgemäßen zuckerhaltigen Diole sind partiell oder total depolymerisierte, trisubstituierte Polysaccharide, vorzugsweise trisubstituierte veretherte Galactomannane, hergestellt nach an sich bekannten Methoden. Die Depolymerisation erfolgt entweder durch saure Hydrolyse und/oder durch Kavitation (Homogenisator, Ultraschall) und/oder durch Scherung (Ultraturrax). Durch Auswahl der Ausgangssubstanz sowie der Reaktionsbedingungen erhält man Oligomere unterschiedlicher Molekulargewichte.

Für die Derivatisierung der erfindungsgemäßen zuckerhaltigen Diole können zur Erniedrigung der mittleren OH-Funktionalität Monoisocyanate wie z.B. Methylisocyanat, Butylisocyanat, Cyclohexylisocyanat, Stearylisocyanat oder Phenylisocyanat eingesetzt werden.

Als Polyisocyanatkomponenten kommen in der Regel Verbindungen der folgenden Formel in Frage

$Q(NCO)_n$ ,

in der

n 2 bis 4, vorzugsweise 2 bedeutet, und

Q einen aliphatischen Kohlenwasserstoffrest mit 2 bis 18, vorzugsweise mit 6 bis 10 C-Atomen, einen aromatischen Kohlenwasserstoffrest mit 6 bis 15, vorzugsweise 6 bis 13 C-Atomen oder einen araliphatischen Kohlenwasserstoffrest mit 8 bis 15, vorzugsweise 8 bis 13 C-Atomen, bedeutet.

Als derartige Polyisocyanate seien beispielsweise genannt:
Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethylcyclohexan, Hexahydro-1,3- und/oder -1,4-phenylendiisocyanat, Perhydro-2,4'- und/oder -4,4'-diphenylmethandiisocyanat.

Bevorzugte aromatische Polyisocyanate sind beispielsweise in DE-OS 2 160 587 (GB-PS 1 368 382) beschrieben. Besonders bevorzugt sind Lysinesterdiisocyanat, Lysinestertriisocyanat, Hexamethylendiisocyanat, 1-Isocyanato-3,3,5-trimethyl-5-isocyanatomethyl-cyclohexan (Isophorondiisocyanat, abgekürzt: IPDI) und 4,4'-Diisocyanato-Dicyclohexylmethan. Geeignete höhermolekulare Polyisocyanate sind Modifizierungsprodukte derartiger einfacher Polyisocyanate, d.h. Polyisocyanate mit z.B. Isocyanurat-, Carbodiimid-, Allophanat-, Biuret- oder Uretdion-Struktureinheiten, wie sie nach an sich bekannten Verfahren aus den beispielhaft genannten einfachen Polyisocyanaten der oben genannten allgemeinen Formel hergestellt werden können. Unter den höhermolekularen, modifizierten Polyisocyanaten sind insbesondere die aus der Polyurethanchemie bekannten Prepolymeren mit endständigen Isocyanatgruppen des Molekulargewichtsbereichs 400 bis 10.000, vorzugsweise 600 bis 8.000 und insbesondere 800 bis 5.000 von Interesse.

Diese Verbindungen werden in an sich bekannter Weise durch Umsetzung von überschüssigen Mengen an einfachen Polyisocyanaten der beispielhaft genannten Art mit organischen Verbindungen mit mindestens zwei gegenüber Isocyanatgruppen reaktionsfähigen Gruppen, insbesondere organischen Polyhydroxylverbindungen hergestellt.

Geeignete derartige Polyhydroxylverbindungen sind sowohl einfache mehrwertige Alkohole des Molekulargewichtsbereichs 62 bis 599, vorzugsweise 62 bis 200, wie z.B. Ethylenglykol, Trimethylolpropan, Propandiol-1,2 oder Butandiol-1,2, insbesondere jedoch höhermolekulare Polyetherpolyole und/oder Polyesterpolyole der aus der Polyurethanchemie an sich bekannten Art mit Molekulargewichten von 600 bis 8.000, vorzugsweise 800 bis 4.000, die mindestens zwei, in der Regel 2 bis 6, vorzugsweise 2 bis 3, primäre und/oder sekundäre Hydroxylgruppen aufweisen.

Selbstverständlich können auch solche NCO-Prepolymere eingesetzt werden, die beispielsweise aus niedermolekularen Polyisocyanaten der beispielhaft genannten Art und Verbindungen mit gegenüber Isocyanatgruppen reaktionsfähigen Gruppen wie z.B. Polythioetherpolyolen, Hydroxylgruppen aufweisenden Polyacetalen, Polyhydroxypolycarbonaten, Hydroxylgruppen aufweisenden Polyesteramiden oder Hydroxylgruppen aufweisenden Copolymerisaten olefinisch ungesättigter Verbindungen erhalten worden sind.

Zur Herstellung der NCO-Prepolymeren geeignete Verbindungen mit gegenüber Isocyanatgruppen reaktionsfähigen Gruppen, insbesondere Hydroxylgruppen, sind beispielsweise die in US-PS 4 218 543, Kolonne 7, Zeile 29 bis Kolonne 9, Zeile 25, beispielhaft offenbarten Verbindungen. Bei der Herstellung der

NCO-Prepolymeren werden diese Verbindungen mit gegenüber Isocyanatgruppen reaktionsfähigen Gruppen mit einfachen Polyisocyanaten der oben beispielhaft genannten Art unter Einhaltung eines NCO/OH-Äquivalentverhältnisses von ca. 1,5:1 bis 20:1, vorzugsweise 5:1 bis 15:1, zur Umsetzung gebracht. Die NCO-Prepolymeren weisen im allgemeinen einen NCO-Gehalt von 2,5 bis 25, vorzugsweise 6 bis 22 Gew.-%, auf. Hieraus geht bereits hervor, daß im Rahmen der vorliegenden Erfindung unter "NCO-Prepolymeren" bzw. unter "Prepolymeren mit endständigen Isocyanatgruppen" sowohl die Umsetzungsprodukte als solche als auch ihre Gemische mit überschüssigen Mengen an nicht umgesetzten Ausgangspolyisocyanaten, die oft auch als "Semiprepolymere" bezeichnet werden, zu verstehen sind.

Zu den gegebenenfalls mitzuverwendenden Hilfs- und Zusatzstoffen zählen beispielsweise Weichmacher, Aromastoffe, Süßmittel, Füllstoffe, z.B. Talkum und Kalciumcarbonat, lösliche Farbstoffe und Pigmente.

Die Füllstoffe werden, falls überhaupt, der Reaktionsmischung vorteilhafterweise in Mengen von bis zu 50 Gew.-%, vorzugsweise bis zu 30 Gew.-%, bezogen auf das Gewicht der Komponenten a) und b), zugegeben (Beispiel 1).

Nähere Angaben über die üblichen Hilfs- und Zusatzstoffe sind der Fachliteratur, beispielsweise der Monographie von J.H. Saunders und K.C. Frisch "High Polymers", Band XVI, Polyurethanes, Teil 2 und 7, Verlag Interscience Publishers 1962 bzw. 1964, zu entnehmen.

Neben den erfindungsgemäßen zuckerhaltigen Diolen können in untergeordneten Mengen des erfindungsgemäßen Systems auch organische Verbindungen mit einem Molekulargewicht von 400 bis 12.000, vorzugsweise 400 bis 6.000, mit 2 bis 5 Hydroxylfunktionen eingesetzt werden. Die hier angegebenen Molekulargewichte sind aus der OH-Zahl der Substanz über folgende Beziehung zu errechnen:

$$\text{Molekulargewicht} = \frac{56.000}{\text{OH-Zahl}} \times \text{Funktionalität}$$

Es können beispielsweise Polyhydroxypolyether der aus der Polyurethanchemie an sich bekannten Art verwendet werden, wie sie durch Alkoxylierung geeigneter Startermoleküle wie Ethylenglykol, Diethylenglykol, 1,4-Dihydroxybutan, 1,6-Dihydroxyhexan, Trimethylolpropan, Glycerin, Pentaerythrit, Sorbit oder Saccharose zugänglich sind. Als Starter können ebenfalls fungieren: Ammoniak oder Amine wie Ethylendiamin, Hexamethylendiamin, 2,4-Diaminotoluol, Anilin oder Aminoalkohole. Die Alkoxylierung erfolgt unter Verwendung von Propylenoxid und/ oder Ethylenoxid in beliebiger Reihenfolge.

Ferner geeignet sind Polyesterpolyole, wie sie durch Umsetzung der beispielhaft genannten niedermolekularen Alkohole mit mehrwertigen Carbonsäuren wie Adipinsäure, Phthalsäure, Hexahydrophthalsäure, Tetrahydrophthalsäure oder den Anhydriden dieser Säuren in an sich bekannter Weise zugänglich sind.

Ebenfalls geeignet sind solche höhermolekularen Polyhydroxypolyether, in denen hochmolekulare Polyaddukte bzw. Polykondensate oder Polymerisate in feindisperser, gelöster oder aufgepfropfter Form vorliegen. Derartige modifizierte Polyhydroxylverbindungen werden z.B. erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) in situ in den Hydroxylgruppen aufweisenden Verbindungen ablaufen läßt. Derartige Verfahren sind beispielsweise in DE-AS 1 168 075 beschrieben. Es ist aber auch möglich, gemäß US-P-3 869 413 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z.B. durch Polymerisation von Styrol und Acrylnitril in Gegenwart von Polyethern (US-P-3 383 351) oder Polycarbonatpolyolen (US-P-3 637 909) erhalten werden, sind für das erfindungsgemäße Verfahren als Zusätze zur Komponente b) geeignet.

Beim erfindungsgemäßen Verfahren können Amine mitverwendet werden. Bevorzugte Amine haben ein Molekulargewicht von 60 bis 300.

Besonders bevorzugt sind aliphatische Diamine wie Isophorondiamin, Bis-(4-aminocyclohexyl)-methan, 1,4-Diaminocyclohexan, Ethylendiamin und seine Homologen, Piperazin und Lysinester.

Bevorzugte niedermolekulare Polyole sind mehrwertige Alkohole mit einem Molekulargewichtsbereich von 62 bis 400, wie die bereits als Startermoleküle erwähnten Substanzen wie Ethylenglykol, Diethylenglykol, 2-Ethyl-2-butyl-1,3-propandiol, 2,2-Diethyl-1,3-propandiol, 1,4-Dihydroxybutan, 1,6-Dihydroxyhexan, Trimethylolpropan, Glycerin, Pentaerythrit, Sorbit oder Dianhydrosorbit. Besonders bevorzugt sind mehrwertige

Alkohole mit einer Funktionalität von 2.

Bevorzugte erfindungsgemäße Katalysatoren sind tert. Amine wie Diazabicyclooctan und Katalysatoren der folgenden Struktur:

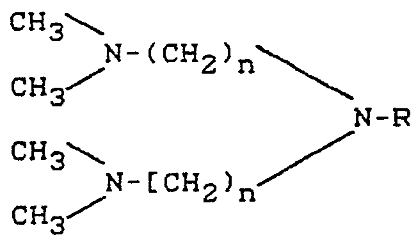

mit n = 2 oder 3 und R = H, CHO oder $CH_3$.

Zur Modifizierung der filmbildenden und Lösungseigenschaften der Polymere sind insbesondere aliphatische verzweigte Diole geeignet, wie 2-Ethyl-2-butyl-1,3-propandiol oder 2,2-Diethyl-1,3-propandiol.

Die für die Synthesen der erfindungsgemäßen Polymere eingesetzten Lösungsmittel sind vorwiegend Dimethylformamid, Dimethylacetamid und DMSO.

Durch die in der pharmazeutischen Technologie bekannten Methoden und Verfahren zum Überziehen von Arzneiformen oder Einbetten von therapeutischen Wirkstoffen erhält man erfindungsgemäße Umhüllungen oder Einbettungen zur Freisetzung dieser Wirkstoffe im Dickdarm.

## Beispiele

### Beispiel 1

Einer Lösung aus 16,00 g (0,1 Mol) 2-Ethyl-2-butyl-1,3-propandiol in 30 ml Dimethylacetamid wird unter vermindertem Druck bei 50° C Wasser entzogen. Man gibt eine Lösung von 25,23 g (0,15 Mol) Hexamethylendiisocyanat in 10 ml Dimethylacetamid zu und rührt das Gemisch unter Stickstoff 48 h bei 80° C. Nach Abkühlen erhält man eine klare Lösung des NCO-Prepolymers [Komponente a)].

Man stellt durch Erwärmen auf 80° C eine Lösung von 17,12 g (0,05 Mol) wasserfreier 1,4-(D-Pyrano-D-pyranose) in 20 ml Dimethylacetamid [Komponente b)] her und gibt diese Lösung bei Raumtemperatur zu der Komponente a). Nach 24 h wird die viskose Lösung in Wasser gefällt und das weiße Präzipitat in Methanol gelöst und erneut in Wasser gefällt. Danach wird es 2 h lang bei 120° C getrocknet.

Das so entstandene Polymer ist löslich in Dimethylsulfoxid, Dimethylacetamid und Methanol; es ist unlöslich in Wasser und Chloroform. Aus einer Lösung des Polymers in Methanol läßt sich ein klarer, elastischer Film gießen.

### Beispiel 2

Eine Lösung von 20,00 g Ethylgalactomannan (DS = 3) in 1000 ml Methanol wird mit 20 ml verdünnter Salzsäure versetzt und 4 h lang bei 50° C gerührt. Das Reaktionsgemisch wird mit methanolischem Natriumhydroxid neutralisiert und das Lösungsmittel unter Vakuum entfernt. Der Rückstand wird in Aceton suspendiert und filtriert. Die in Aceton gelösten Ethylgalactomannanoligomere werden durch Verdampfen des Lösungsmittels unter vermindertem Druck isoliert.

Analog Beispiel 1 wird dann ein Polymer aus 13,22 g (0,1 Mol) 2,2-Diethyl-1,3-propandiol, 25,23 g (0,15 Mol) Hexamethylendiisocyanat und 4,96 g an Ethylgalactomannanoligomeren hergestellt.

Das so entstandene Polymer ist löslich in Dimethylsulfoxid, Dimethylacetamid, Aceton, Methanol, Chlorofom und Dichlormethan; es ist unlöslich in Wasser. Aus einer Lösung des Polymers in Chloroform läßt sich ein klarer, elastischer Film gießen.

### Beispiel 3

Eine Lösung von 20,00 g tris-Phenylcarbamatgalactomannan in 500 ml 98 %iger Essigsäure wird mit 10 ml Acetanhydrid und 3,5 ml konzentrierter Schwefelsäure versetzt und 5 h bei 80° C unter Rühren gelassen. Das Reaktionsgemisch wird in Petroleumbenzin gefällt, der Rückstand abfiltriert und mit Wasser gewaschen, bis er neutral reagiert.

Danach bestimmt man nach üblicher Methode die Menge der substituierten Galactomannanoligomere, welche mit 5,95 g (0,05 Mol) Phenylisocyanat äquivalent ist (OH-Zahl).

Analog Beispiel 1 wird dann ein Polymer aus 13,22 g (0,1 Mol) 2,2-Diethyl-1,3-propandiol, 25,23 g (0,15 Mol) Hexamethylendiisocyanat und der berechneten Menge an Carbamatgalactomannanoligomeren hergestellt.

Das so entstandene Polymer ist löslich in Dimethylsulfoxid, Dimethylacetamid, Aceton, Methanol; es ist unlöslich in Wasser. Aus einer Lösung des Polymers in Aceton läßt sich ein klarer, elastischer Film gießen.

Beispiel 4

Eine Lösung von 20,00 g Galactomannantriacetat in 500 ml 98 %iger Essigsäure wird mit 10 ml Acetanhydrid und 3,5 ml konzentrierter Schwefelsäure versetzt und 5 h bei 80°C unter Rühren gelassen. Das Reaktionsgemisch wird in Petroleumbenzin gefällt, der Rückstand abfiltriert und mit Wasser gewaschen, bis er neutral reagiert.

Danach bestimmt man nach üblichen Methoden die Oligomerenmenge des Galactomannantriacetats, welche mit 5,95 g (0,05 Mol) Phenylisocyanat äquivalent ist (OH-Zahl).

Analog Beispiel 1 wird dann ein Polymer aus 13,22 g (0,1 Mo) 2,2-Diethyl-1,3-propandiol, 25,23 g (0,15 Mol) Hexamethylendiisocyanat und 4,96 g an Galactomannanacetatoligomeren hergestellt.

Das so entstandene Polymer ist löslich in Dimethylsulfoxid, Dimethylacetamid, Aceton, Methanol; es ist unlöslich in Wasser. Aus einer Lösung des Polymers in Aceton läßt sich ein klarer, elastischer Film gießen.

Beispiel 5

Analog Beispiel 1 wird ein Polymer aus 32,00 g (0,2 Mol) 2-Ethyl-2-butyl-1,3-propandiol, 50,46 g (0,30 Mol) Hexamethylendiisocyanat und 4,96 g an depolymerisiertem Phenylcarbamatgalactomannan sowie 4,96 g an depolymerisiertem Galactomannantriacetat, hergestellt.

Das so entstandene Polymer ist löslich in Dimethylsulfoxid, Dimethylacetamid und Methanol; es ist unlöslich in Wasser. Aus einer Lösung des Polymers in Methanol läßt sich ein klarer, elastischer Film gießen.

Beispiel 6

Eine erforderliche Menge Arzneistoff (in der Regel zwischen 5 und 10 Gewichtsteile) wird mit 46,5 bis 51,5 Gewichtsteilen Lactose, 30 Gewichtsteilen Maisstärke und 1 Gewichtsteil quervernetztem PVP vermischt und durch Zugabe von 8 Gewichtsteilen einer 20 %igen Lösung von PVP 25 in Wasser, nach bekannten Verfahren granuliert. Das entstandene Granulat wird getrocknet und anschließend mit einer Gleit- und Sprengmittelmischung aus 2 Gewichtsteilen Talkum, 3 Gewichtsteilen mikrokristalliner Cellulose, 0,5 Gewichtsteilen Magnesiumstearat und 3 Gewichtsteilen quervernetztem PVP vermischt. Anschließend wird diese Mischung mit einer Tablettenpresse zu Tablettenkernen mit einem Durchmesser von 6 mm, einer Gesamthöhe von 2,9 mm und einer Steghöhe von 2,1 mm tablettiert.

Die so hergestellten Kerne werden mit Hilfe eines Wirbelschicht-Umhüllungsverfahrens mit dem erfindungsgemäßen Polymer überzogen, indem eine 5 %ige Lösung des Polymers in Aceton, mit einer geeigneten Sprührate (nicht zu langsam und nicht zu schnell, damit keine Überfeuchtungen auftreten), auf die Tablettenkerne gesprüht wird. Hierzu wird die Gesamtmenge der aufgesprühten 5 %igen Lösung so gewählt, daß bei theoretischer Ausbeute 100 g Lösung gerade 100 g Kernen entsprechen. Die fertige Umhüllung hat dann eine Schichtdicke von 50 $\mu$m.

Die auf dieser Weise umhüllten Kerne zerfallen weder in künstlichem Magensaft (Prüfung nach DAB 9 oder Ph. Eur.), noch innerhalb von 5 Stunden in künstlichem Dünndarmsaft (hergestellt nach USP XXI). Nach Einbringen der so umhüllten Kerne in eine Kultur anaerobischer Dickdarmbakterien, erfolgt der Zerfall und die Wirkstofffreisetzung innerhalb von 1 Stunde, d. h. nachdem die glycolytischen Enzyme der Dickdarm-Mikroflora die erfindungsgemaße polymere Umhüllung gespalten haben.

Beispiel 7

0,5 Gewichtsteile eines peptidischen Arzneistoffs (z. B. Ocitocin, Somatostatin, ACTH, Insulin etc.) in kristalliner bzw. fester Form werden in 40 Gewichtsteilen einer 7,5 %igen Lösung eines erfindungsgemäßen Polymers in Aceton unter Rühren fein suspendiert. Anschließend wird die Suspension nach bekannten Verfahren sprühgetrocknet, so daß sich nach dem Verdunsten des Lösungsmittels ein Film des Polymers

auf den Oberflächen der suspendierten Partikel niederschlägt. Die so erhaltenen Mikrokapseln werden mit Wasser gewaschen und getrocknet. Danach werden sie in Hartgelatinekapseln abgefüllt (0,02 g - 0,25 g Mikrokapseln pro Hartgelatinekapsel).

Die mit diesen Mikrokapseln gefüllten Hartgelatinekapseln zerfallen innerhalb von 20 Minuten in künstlichem Magensaft (Prüfung nach DAB 9 bzw. Ph. Eur.) und setzen die Mikrokapseln frei. Die freigesetzten Mikrokapseln zerfallen weder in künstlichem Magensaft (Prüfung nach DAB 9 bzw. Ph. Eur.) noch innerhalb von 5 Stunden in künstlichem Dünndarmsaft (hergestellt nach USP XXI). Der Zerfall und die Wirkstofffreisetzung der Mikrokapseln erfolgen jedoch innerhalb von 1 Stunde, nachdem die Mikrokapseln in eine Kultur anaerobischer Dickdarmbakterien eingebracht wurden. Hierbei spalten die glycolytischen Enzyme der Dickdarm-Mikroflora die erfindungsgemäße polymere Umhüllung.

Beispiel 8

25 Gewichtsteile einer 2 %igen wäßrigen Lösung eines peptidischen Arzneistoffes (z. B. Ocitocin, Somatostatin, ACTH, Insulin etc.) werden in 40 Gewichtseile einer 7,5 %igen Lösung eines erfindungsgemäßen Polymers in Aceton unter schnellem Rühren langsam eingetropft. Auf diese Weise wird der Wirkstoff fein dispers in eine polymere Matrix eingebettet.

Die ausgefallenen wirkstoffhaltigen Partikel dieser Polymermatrix werden abfiltriert, mit Wasser gewaschen und getrocknet. Die so erhaltenen Einbettungspartikel werden in Hartgelatinekapseln abgefüllt (0,02 g - 0,25 g Einbettung pro Hartgelatinekapsel).

Anschließend werden die Hartgelatinekapseln mit Hilfe eines Wirbelschicht-Umhüllungsverfahrens mit einem bekannten magensaftresistenten Filmüberzug (z. B. EUDRAGIT® L,EUDRAGIT® S, CAP) umhüllt.

Die abgefüllten Hartgelatinekapseln zerfallen nicht in künstlichem Magensaft (Prüfung nach DAB 9 bzw. Ph. Eur.) sondern innerhalb von 60 Minuten in künstlicher Dünndarmflüssigkeit (hergestellt nach USP XXI) und setzen die Polymerpartikel frei. Die Polymermatrix-Partikel zerfallen nicht innerhalb von 5 Stunden in künstlicher Dünndarmflüssigkeit (hergestellt nach USP XXI). Der Zerfall und die Wirkstofffreisetzung erfolgt innerhalb 1 Stunde, erst nachdem die Partikel in eine Kultur anaerobischer Dickdarmbakterien eingebracht wurden. Hierbei spalten die glycolytischen Enzyme der Dickdarm-Mikroflora die erfindungsgemäße Polymermatrix.

Beispiel 9

100 Gewichtsteile Metoprolol werden in einer Lösung von 150 Gewichtsteilen eines erfindungsgemäßen Polymers in 2000 - 3000 Gewichtsteilen Aceton suspendiert bzw. gelöst und diese Lösung in einer geeigneten Sprühtrocknungsanlage zersprüht und getrocknet. Dabei wird der Arzneistoff in den sprühgetrockneten Polymerpartikeln feinst dispers eingebettet.

Diese Partikel können zu Tabletten oder Kapseln weiter verarbeitet werden. Die Tabletten oder Kapseln können zusätzlich auch noch in üblicher Weise magensaftresistent lackiert werden.

## Patentansprüche

1. Zuckerhaltige Polymere zur Umhüllung und/oder Einbettung von oral applizierbaren pharmazeutischen Wirkstoffen, enthaltend Di- und/oder Polysaccharidsegmente, die erst im Dickdarm die enthaltenden Wirkstoffe freigeben.

2. Mit Di- und/oder Polysaccharidsegmente aufweisenden zuckerhaltigen Polymeren umhüllte und/oder in solche Polymere eingebettete oral applizierte pharmazeutische Wirkstoffe.

3. Pharmazeutische Zubereitungen gemäß Anspruch 2, dadurch gekennzeichnet, daß die zuckerhaltigen Polymeren Urethan- und/oder Harnstoff-Strukturen aufweisende Block-Copolymere darstellen.

4. Pharmazeutische Zubereitungen gemäß Ansprüchen 2 und 3, dadurch gekennzeichnet, daß die zuckerhaltigen Polymeren als Saccharidsegmente lineare oder verzweigte Kohlenhydrate vom Molekulargewicht 340 bis ca. 10.000 enthalten.

5. Pharmazeutische Zubereitungen gemäß Ansprüchen 2 bis 4, dadurch gekennzeichnet, daß in den zuckerhaltigen Polymeren die Di- oder Polysaccharidsegmente als Pyranoseeinheiten vorliegen, wobei pro Pyranoseeinheit bis zu drei OH-Gruppen substituiert sind.

6. Pharmazeutische Zubereitungen gemäß Ansprüchen 2 bis 5, dadurch gekennzeichnet, daß in den zuckerhaltigen Polymeren die Pyranoseeinheiten mit einem Substitutionsgrad von 2,5 bis 3 vorliegen.

7. Pharmazeutische Zubereitungen gemäß Ansprüchen 5 und 6, dadurch gekennzeichnet, daß in den zuckerhaltigen Polymeren die Pyranoseeinheiten durch aliphatische, araliphatische und/oder aromatische Ether, Ester und/oder Carbamate substituiert sind.

8. Pharmazeutische Zubereitungen gemäß Ansprüchen 2 bis 5, dadurch gekennzeichnet, daß die zuckerhaltigen Polymeren unter Verwendung von aliphatischen, araliphatischen und/oder aromatischen Polyisocyanaten aufgebaut sind.

9. Verfahren zur Herstellung von Di- und/oder Polysaccharidsegmente aufweisenden zuckerhaltigen Polymeren gemäß Anspruch 1, dadurch gekennzeichnet, daß man eine überschüssige Menge eines Polyisocyanats mit Di- und/oder Polysaccharidsegmente aufweisenden zuckerhaltigen Diolen, gegebenenfalls im Gemisch mit nicht-zuckerhaltigen Polyolen des Molgewichtsbereichs 62 bis 12.000, zu endständige Isocyanatgruppen aufweisenden Prepolymeren umsetzt, anschließend eine Kettenverlängerung dieser Prepolymeren mit Kettenverlängerungsmitteln durchführt.

10. Verfahren zur Herstellung von mit Di- und/oder Polysaccharidsegmente aufweisenden zuckerhaltigen Polymeren umhüllte und/oder in solche Polymere eingebettete oral applizierbaren pharmazeutischen Wirkstoffen gemäß Anspruch 2 bis 8, dadurch gekennzeichnet, daß man eine überschüssige Menge eines Polyisocyanats mit Di- und/oder Polysaccharidsegmente aufweisenden zuckerhaltigen Diolen, gegebenenfalls im Gemisch mit nicht-zuckerhaltigen Polyolen des Molgewichtsbereichs 62 bis 12.000, zu endständige Isocyanatgruppen aufweisenden Prepolymeren umsetzt, anschließend eine Kettenverlängerung dieser Prepolymeren mit Kettenverlängerungsmitteln durchführt und pharmazeutische Wirkstoffe mit den so erhaltenen Urethan- und/oder Harnstoff-Strukturen aufweisenden zuckerhaltigen Polymeren umhüllt und/oder in diese Polymeren einbettet.

11. Verfahren gemäß Ansprüchen 9 und 10, dadurch gekennzeichnet, daß man als Polyisocyanate aliphatische und/oder cycloaliphatische und/oder aromatische Diisocyanate der Formel

$Q\,(NCO)_n$

in der

n 2 bis 4, vorzugsweise 2, bedeutet und

Q einen Kohlenwasserstoffrest mit 2 bis 18, vorzugsweise 6 bis 10 C-Atomen, darstellt, verwendet.

12. Verfahren gemäß Ansprüchen 9 und 10, dadurch gekennzeichnet, daß man als Polyisocyanate Lysinester-diisocyanate und/oder Lysinester-triisocyanat verwendet.

13. Verfahren gemäß Ansprüchen 9 und 10, dadurch gekennzeichnet, daß man als Polyisocyanate Estergruppen aufweisende aromatische Polyisocyanate mit den Struktureinheiten der Formel

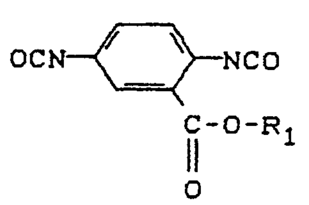

und/oder

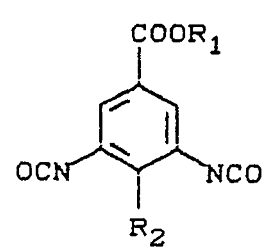

worin

$R_1$ einen gegebenenfalls verzweigten $C_1$-$C_{20}$-, vorzugsweise $C_1$-$C_8$-Alkylrest und

$R_2$ einen $C_1$-$C_{10}$-, vorzugsweise $C_1$-$C_4$-Alkylrest, $C_1$-$C_4$-Alkoxygruppen, vorzugsweise eine O-$CH_3$-Gruppe oder ein Halogenatom, vorzugsweise ein Chloratom bedeuten, verwendet.

**14.** Verfahren gemäß Ansprüchen 9 und 10, dadurch gekennzeichnet, daß man Mischungen unterschiedlicher Polyisocyanate verwendet.

**15.** Verwendung von Di- und/oder Polysaccharidsegmente aufweisenden zuckerhaltigen Polymeren gemäß Anspruch 1, gegebenenfalls in Gegenwart von Hilfs- und Zusatzstoffen zur Herstellung von Filmüberzügen und Einbettungen von oral applizierbaren pharmazeutischen Wirkstoffen.

**16.** Oral applizierbare Arzneizubereitungen mit einer Wirkstoff-Freigabe im Dickdarm, dadurch gekennzeichnet, daß die Wirkstoffe von Di- und/oder Polysaccharidsegmente aufweisenden zuckerhaltigen Polymeren umhüllt und/oder in diese eingebettet sind.

Europäisches Patentamt

# EUROPÄISCHER RECHERCHENBERICHT

Nummer der Anmeldung

**EP 90 12 4695**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 095 968 (C.N.R.S.)<br>* Ansprüche 1,5-7; Seite 3, Zeilen 1-25; Seite 5, Zeile 29 - Seite 6, Zeile 5; Seite 7, Zeile 29 - Seite 8, Zeile 12 * | 1-5,8, 15-16 | C 08 G 18/32<br>C 08 G 18/64<br>A 61 K 9/20<br>A 61 K 9/32<br>A 61 K 9/50<br>A 61 K 9/16 |
| X | MACROMOLECULAR SYNTHESES, Band 7, 1979, Seiten 101-105, New York, US; S. KIM et al.: "Copolymers of depolymerized-cellulose triacetate and diisocyanates"<br>* Seite 101, Formel; Seite 102, Absätze 1-3 * | 1,3-9 | |
| X | DIE MAKROMOLEKULARE CHEMIE, Band 181, Nr. 9, 1980, Seiten 1861-1870, Heidelberg, DE; K. KURITA et al.: "Synthesis and properties of polyurethanes from D-cellobiose and diisocyanates"<br>* Seite 1862, Absatz 4 - Seite 1865, Absatz 1 * | 1,3,4,8 | |
| D,X | US-A-3 950 282 (R.D. GILBERT et al.)<br>* Ansprüche 1-4; Spalte 4, Zeilen 30-43; Spalte 5, Zeile 33 - Spalte 6, Zeile 28; Spalte 7, Zeilen 30-48; Spalte 9, Zeilen 3-56 * | 1-8 | |
| D,X | US-A-3 386 932 (H.W. STEINMANN)<br>* Ansprüche 1-6,19-22; Spalte 12, Zeile 75 - Spalte 13, Zeile 20; Spalte 14, Zeilen 28-51; Spalte 16, Zeilen 9-28 * | 1-11 | **RECHERCHIERTE SACHGEBIETE (Int. Cl.5)**<br>C 08 G<br>A 61 K |
| X | DE-A-2 038 752 (WISCONSIN ALUMNI RESEARCH)<br>* Ansprüche 1-7; Seite 8, Absatz 3 * | 1-8 | |

Der vorliegende Recherchenbericht wurde für alle Patentansprüche erstellt

| Recherchenort | Abschlußdatum der Recherche | Prüfer |
|---|---|---|
| Den Haag | 18 Juni 91 | VAN PUYMBROECK M.A. |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument